(19) Europäisches Patentamt
European Patent Office
Office européen des brevets

(11) Publication number: 0 029 364 A1

# (12) EUROPEAN PATENT APPLICATION

(21) Application number: 80304107.8

(22) Date of filing: 14.11.80

(51) Int. Cl.³: C 07 D 231/14, A 61 K 31/415

(30) Priority: 16.11.79 JP 149103/79

(43) Date of publication of application: 27.05.81 Bulletin 81/21

(84) Designated Contracting States: AT BE CH DE FR GB IT LI NL SE

(71) Applicant: MORISHITA PHARMACEUTICAL CO. LTD., 29 Doshomachi 4-chome Higashi-ku, Osaka (JP)

(72) Inventor: Seki, Kunio, 1823-1 Ohshinohara, Yasu-cho Yasu-gun, Shiga-ken (JP)
Inventor: Ohki, Masahiko, 720-77 Toba-Otsu, Yasu-cho Yasu-gun, Shiga-ken (JP)

(74) Representative: Myerscough, Philip Boyd et al, J.A.Kemp & Co. 14, South Square Gray's Inn, London, WC1R 5EU (GB)

## (54) 5-Alkylpyrazol-3-carboxylic acid derivatives for use in therapy.

(57) Derivatives of pyrazole of the general formula:

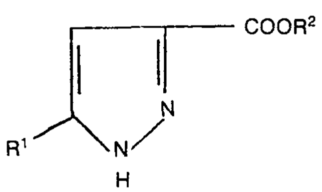

(I)

wherein $R^1$ represents an alkyl group of 7 to 11 carbon atoms and $R^2$ represents a hydrogen atom or a lower alkyl group, have therapeutic activity, in particular in the treatment of hyperlipemia, and have very low mammalian toxicity.

COMPLETE DOCUMENT

EP 0 029 364 A1

0029364



DESCRIPTION

"5-ALKYLPYRAZOL-3-CARBOXYLIC ACID DERIVATIVES FOR USE IN THERAPY"

The present invention relates to derivatives of pyrazole for use in therapy, in particular in the treatment of hyperlipemia.

Hitherto, various antihyperlipemic pharmaceutical compositions have been proposed for treatment of hyperlipemia, but they cause side effects during the long term administration to the patients. For instance, clofibrate (2-(4-chlorophenoxy)-2-methylpropanoic acid ethyl ester) which has been used as an antihyperlipemic pharmaceutical composition causes diseases in the bile duct system and is in danger of causing cancer, so that clinical administration of clofibrate has been restricted. Pharmaceutical compositions containing nicotinic acid as an active ingredient for treating hyperlipemia are rarely used because of the side effect of causing flushing or eruptions on the face and skin.

5-Methylpyrazol-3-carboxylic acid was reported to reduce the level of serum lipid (refer to Kupiecki, F. et al., J. pharmacol. Expt. Therap., 160 (1), 166, 1968), but it was shown later that in addition to an uncertainty as to its effectiveness in the treatment of hyperlipemia there was a side effect of causing a reduction in blood sugar-level, and accordingly it was not considered suitable for use as an active ingredient in a pharmaceutical composition for treating hyperlipemia (refer to Lang, P. D. et al., Arzneim. Forsch., 25 (1), 117, 1975).

After considering the present situation of antilipemic agents and after synthesizing and testing

various compounds, we have found that certain derivatives of pyrazole have an excellent antilipemic activity and do not exhibit noticeable side effects.

The present invention thus provides a compound for use in a method of treatment of the human or animal body by therapy, wherein the compound is of the formula (I):

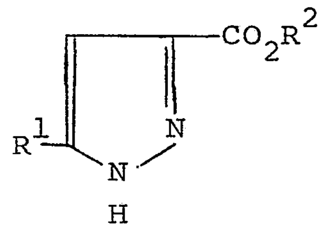

(I)

wherein $R^1$ represents an alkyl group of 7 to 11 carbon atoms and $R^2$ represents a hydrogen atom or a lower (preferably $C_1$ to $C_4$) alkyl group.

Although formula (I) includes within its scope known compounds (refer to Keskin et al., Istanbul University, Fen. Fak. Mecum. Setic., 34,95-108, 1969), we are aware of no disclosure concerning the pharmacological properties of the pyrazole derivatives of formula (I).

The pyrazole derivatives of formula (I) can be synthesized by the following method:

A sodium enolate of the general formula (II):

$$R^1\text{-COCH} = \underset{\substack{| \\ O\ -\ Na}}{C} - CO_2C_2H_5 \qquad \text{(II)}$$

wherein $R_1$ is as defined above, which is obtainable by Claisen's condensation of higher-alkyl methyl ketone

and diethyl oxalate, is heated in a mineral acid with hydrazine hydrate under reflux for 4 to 10 hours with stirring to obtain an ethyl ester of 5-alkylpyrazole carboxylic acid.

In the next step, the thus obtained ester is hydrolyzed with sodium hydroxide in an aqueous lower alcoholic solution at about 100°C to obtain the 5-alkylpyrazole-3-carboxylic acid.

Alternatively, by heating the sodium enolate of formula (II) with hydrazine sulphate in an aqueous lower alcoholic solution of sodium hydroxide under reflux for 2 to 3 hours, 5-alkylpyrazole-3-carboxylic acids are obtained.

Methods for synthesizing compounds of formula (I) and their physical- and chemical properties are described in Examples 1 to 6 below. The toxicological and pharmacological properties of the compounds of these Examples will first be described as follows:

1. Toxicological property

Test method for acute toxicity:

Male ICR mice of body weight of 21 to 25 g were divided into several groups each consisting of 8 animals and were fasted for 17 to 18 hours and then given orally a compound of formula (I) or nicotinic acid dissolved or dispersed in an aqueous 1% carboxymethyl cellulose (CMC) solution at a rate of 0.2 mℓ/10 g body weight. The administered levels of each compound were 2.5 g/kg body weight and 5.0 g/kg, and from the mortalities at both levels, $LD_{50}$ was roughly calculated and compared with $LD_{50}$ of nicotinic acid.

The results showed that each compound of formula (I) tested had a $LD_{50}$ value over 5.0 g/kg body weight which was much larger than that of nicotinic acid, 4.5 g/kg.

2. Pharmacological property

Pharmacological tests were carried out on the compounds of Examples 1 to 6 using nicotinic acid as a reference.

(i) Pharmacological test against hypertriglyceridemia:

Seven groups of male SD rats each weighing 232 - 237 g, each group consisting of 6 animals, were used for the test animals and one group consisting of 12 animals was used as control. The test rats were made to be hypertriglyceridemic by giving them an aqueous 10% by weight solution of fructose for 2 days, while the rats of the control group were given tap water.

The compounds of Examples 1 to 6 and nicotinic acid were each separately dissolved or dispersed into an aqueous 1% solution of CMC at a predetermined concentration, and the thus prepared solution or dispersion was orally administered in three doses of 0.5 mℓ/100 g body weight, a first dose just after the commencement of the test, a second dose 24 hours after the first administration, and a third dose 18 hours after the second administration. The dose of each compound was set so that each animal received 300 mg/kg of each compound as free carboxylic acid. The rats of the control group were given an aqueous 1% solution of CMC only in three doses at the same intervals as the test groups. All the groups were fed with the same diet during the test period.

After 48 hours of the commencement of the test, blood samples were collected from each animal by amputation of the carotid artery, and serum was separated from each blood sample and frozen to be kept until lipid analysis.

Lipid analysis:

Serum triglyceride was determined by the Acetylacetone Method, and the reduction percentage of

lipid was calculated by the following formula:

$$\left(1 - \frac{\text{amount of triglyceride in serum of test animal}}{\text{amount of triglyceride in serum of control}}\right) \times 100$$

= reduction percentage of lipid

wherein the amount for each group is the average for the test animals in the group.

Analysis for serum cholesterol:

The content of serum cholesterol was determined by an enzymatic method using cholesterol-oxidase, and the reduction percentage of cholesterol was calculated by the following formula:

$$\left(1 - \frac{\text{amount of cholesterol in serum of test animal}}{\text{amount of cholesterol in serum of control}}\right) \times 100$$

= reduction percentage of cholesterol

The results of the analyses are shown in Table 1.

TABLE 1

| Compound tested | Total Dose (mg/ kg) | Reduction Percentage | |
|---|---|---|---|
| | | Triglyceride (%) | Cholesterol (%) |
| Ethyl 5-n-heptyl-pyrazole-3-carboxylate | 340 | 41.1 | 2.1 |
| Ethyl 5-n-nonyl-pyrazole-3-carboxylate | 335 | 63.1 | 29.5 |
| Ethyl 5-n-undecyl-pyrazole-3-carboxylate | 331 | 83.7 | 53.1 |
| 5-n-heptyl-pyrazole-3-carboxylic acid | 300 | 40.6 | 12.5 |
| 5-n-nonyl-pyrazole-3-carboxylic acid | 300 | 66.5 | 12.2 |
| 5-n-undecyl-pyrazole-3-carboxylic acid | 300 | 69.1 | 40.0 |
| nicotinic acid | 300 | 24.1 | 16.4 |

(ii) Pharmacological test against hypercholesterolemia:

Three groups of male SD rats each weighing ca. 150g, each group consisting of five rats, were used as the test animals and one group consisting of 10 rats was used as control. All animals were fed a high cholesterol diet consisting of 1% cholesterol, 0.2% sodium cholate, 5% olive oil and commercial diet up to total 100% for 7 days.

The test compounds of the present invention and clofibrate, as a medicine for comparison, were respectively dissolved or dispersed in an aqueous 1% solution of CMC so that the volume of the solution or dispersion administered each time to each animal was 0.5 mℓ/100g body weight, and the solution or dispersion was orally administered once a day for 7 consecutive days so that the amount administered each time of each pyrazole derivative or of clofibrate was 200 mg/kg body weight. Water was taken ad lib. After 20 hours of fasting from the last administration, a blood sample was taken from each animal by amputation of the carotid artery and the serum separated was frozen until analysed for cholesterol as follows:

Cholesterol analysis

The content of serum cholesterol was determined by the above-mentioned method. The beta-lipoprotein-cholesterol was determined by "heparin-calcium precipitation method". The reduction percentages of cholesterol in serum and beta-lipoprotein were calculated in the same manner as above. The results are shown in Table 2.

Table 2

| Compound tested | Dose (mg/kg/day) | Reduction Percentage | |
|---|---|---|---|
| | | Cholesterol in serum | Cholesterol in beta-lipoprotein |
| 5-n-Nonyl-pyrazole-3-carboxylic acid | 200 | 11.1 | 0.6 |
| 5-n-Undecyl-pyrazole-3-carboxylic acid | 200 | 22.8 | 20.1 |
| Clofibrate (for comparison) | 200 | -3.5 | -2.0 |

As seen from Tables 1 and 2, pyrazole derivatives of formula (I) have an excellent antilipemic activity in common, which activity is superior to that of clofibrate which has been used as an antilipemic agent for a long time.

The following description is an explanation of formulating pyrazole derivatives of formula (I) into a pharmaceutical composition. In general, the pyrazole derivatives can be formulated into pharmaceutical compositions of dose unit form by known techniques. Since the pyrazole derivatives will generally be

administered orally, they are preferably formulated into tablets or capsules. When formulating the pyrazole derivatives into dose unit form, known vehicles, disintegrating agents, binding agents and lubricating agents, widely used in the pharmaceutical field, may be used.

The dose level of the pyrazole derivatives will naturally depend upon the degree of hyperlipemia, but, in general, a suitable daily oral dose is 0.2 to 3.0 g/60 kg of body weight, preferably 0.5 to 1.5 g/60 kg body weight.

The following Examples provide a detailed explanation of the synthesis (Examples 1 to 6) and the formulation (Examples 7 and 8) of pyrazole derivatives of formula (I).

Example 1

Synthesis of ethyl 5-n-heptylpyrazole-3-carboxylate:

12.6 g (0.55 mol) of metallic sodium was added slowly, with agitation, to 500 ml of absolute ethanol, until dissolved completely, and after cooling, a liquid mixture of 78 g (0.55 mol) of methyl n-heptyl ketone and 80.3 g (0.55 mol) of diethyl oxalate was slowly dropped into the solution at room temperature with agitation. Stirring was continued for 8 hours after the addition, at room temperature. The resulting yellow precipitate was collected by filtration and recrystallized from ethanol to provide sodium 1-ethoxycarbonyl-3-oxo-1-decenolate as pale yellow needle-like crystals in an amount of 117 g corresponding to a yield of 80.0%. After dissolving 26.4 g (0.10 mol) of the sodium decenolate in 23 ml of glacial acetic acid, 5.5 g (0.11 mol) of hydrazine hydrate was dropped slowly into the solution under ice cooling. After the addition of hydrazine hydrate, the mixture was heated under

reflux for 8 hours. Then, the reaction mixture was cooled and poured into iced water, and then neutralized with an aqueous saturated solution of sodium hydrogen carbonate and extracted with benzene. The benzene layer was washed with an aqueous saturated solution of sodium chloride and dried over anhydrous sodium sulphate. After distilling off the solvent under a reduced pressure, the residue was recrystallized from petroleum ether to obtain the desired product as colourless prisms in an amount of 11.5 g corresponding to a yield of 54.8% theoretical.

Elementary analysis :

found : 65.58 % C; 9.35 % H and 11.60 % N

calcd. as $C_{13}H_{22}O_2N_2$ : 65.51 % C; 9.31 % H and 11.76 % N.

Infrared absorption spectrum

$\nu_{max}^{nujol}$ 3300 to 3100 $cm^{-1}$ ($\nu_{NH}$) and 1718 $cm^{-1}$ ($\nu_{C=O}$).

Mass spectrum, m/e : 238 ($M^+$).

Nuclear magnetic resonance spectrum (NMR) (in $CDCl_3$) $\delta$:

0.85 (3H, t, J = 6 Hz, $\underline{CH_3}(CH_2)_6$)

1.2 to 1.5 (11H, $CH_3\underline{(CH_2)_4}(CH_2)_2$ and $\underline{CH_3}CH_2OCO$)

1.65 (2H, m, $CH_3(CH_2)_4 - \underline{CH_2} - CH_2$)

2.72 (2H, t, J = 8 Hz, $CH_3(CH_2)_5 - \underline{CH_2}$)

4.35 (2H, q, J = 7 Hz, $CH_3 - \underline{CH_2} - OCO$)

6.55 (1H, s, H in the pyrazole ring)

11.30 (1H, br., NH which disappears by treatment with $D_2O$)

Example 2:

Synthesis of 5-n-heptylpyrazole-3-carboxylic acid: 20.0 g (0.084 mol) of ethyl 5-n-heptylpyrazole-3-carboxylate obtained as in Example 1 was dissolved in 81 ml of a 5 % methanolic solution of sodium hydroxide, and the solution was heated for 5 hours in a reflux condenser. After cooling the reaction mixture water was added, then methanol was distilled off under a reduced pressure, and the residue was acidified (pH of about 2) with concentrated hydrochloric acid. The deposited white precipitate was collected by filtration and recrystallized from a mixture of distilled water and methanol to obtain 5-n-heptylpyrazole-3-carboxylic acid as colourless needle-like crystals melting at 169 - 171°C in an amount of 13.3 g corresponding to a yield of 75.2 %.

Elementary analysis :

| | |
|---|---|
| found | : 62.71 % C; 8.50 % H and 13.42 % N |
| calcd. as $C_{11}H_{18}O_2N_2$ | : 62.83 % C; 8.63 % H and 13.32 % N. |

Infrared absorption spectrum

$\nu_{max}^{KBr}$ 3240 $cm^{-1}$ (br. $\nu_{OH}$ and NH) , 3000 to 2300 $cm^{-1}$ (br. $\nu_{NH_2^+}$ and $CO_2H$) and 1700 $cm^{-1}$ ($\nu_{C=O}$).

Mass spectrum m/e : 210 ($M^+$).

NMR (in $d_6$-DMSO) $\delta$ :

0.87 (3H, t, J = 7 Hz, $\underline{CH_3}(CH_2)_6$)

1.2 to 1.5 (8H, br. s, $CH_3$ - $\underline{(CH_2)_4}$ - $CH_2CH_2$)

1.65 (2H, m, $CH_3(CH_2)_4$ - $\underline{CH_2}$ -$CH_2$ )

2.61 (2H, t, J = 7 Hz, $CH_3(CH_2)_4$ - $CH_2$ - $\underline{CH_2}$)

6.52 (1H, s, H in the pyrazole ring)
10.65 (2H, br., NH and $CO_2H$ which disappear by treatment with $D_2O$).

Example 3:

Synthesis of ethyl 5-n-nonylpyrazole-3-carboxylate:

After slowly adding and dissolving 5.2 g (0.23 mol) of metallic sodium in 300 ml of absolute ethanol under agitation, the resulting clear solution was cooled and a liquid mixture of 39 g (0.23 mol) of methyl n-nonyl ketone and 33.6 g (0.23 mol) of diethyl oxalate was dropped slowly into the solution at room temperature with agitation. After the addition was complete, the reaction mixture was heated at 60°C with agitation for 5 hours. Then, the reaction mixture was cooled with iced water, and the resulting yellow precipitate was collected by filtration and recrystallized from ethanol to provide sodium 1-ethoxycarbonyl-3-oxo-1-dodecenolate as pale yellow needle-like crystals in an amount of 44 g corresponding to a yield of 65.5 %.

26.0 g (0.09 mol) of the thus obtained dodecenolate was dissolved in 23 ml of glacial acetic acid, and 5.0 g (0.10 mol) of hydrazine hydrate was slowly dropped into the solution with ice cooling and stirring. After the addition was complete, the reaction mixture was heated under reflux for 6 hours. Then, the reaction mixture was cooled and poured into iced water, and was neutralized with a saturated aqueous solution of sodium hydrogen carbonate and extracted with benzene. The benzene layer was isolated, washed with a saturated aqueous solution of sodium chloride and dried with anhydrous sodium sulfate. Then, the dried benzene layer was subjected to

BAD ORIGINAL

distillation under a reduced pressure to remove the solvent, and the residue was recrystallized from n-hexane to provide ethyl 5-n-nonylpyrazole-3-carboxylate as colourless needle-like crystals melting at 37 to 38°C in an amount of 14.5 g corresponding to a yield of 63.4 %.

Elementary analysis :
found : 67.92 % C; 9.80 % H and 10.88 % N
Calcd. as $C_{15}H_{26}O_2N_2$ : 67.63 % of C; 9.84 % H and 10.52 % N.

Infrared absorption spectrum
$\nu_{max}^{nujol}$ 3300 to 3100 $cm^{-1}$ ($\nu_{NH}$) and 1720 $cm^{-1}$ ($\nu_{C=O}$).

Mass spectrum, m/e : 266 ($M^+$).

NMR (in $CDCl_3$) $\delta$ :

0.85 (3H, t, J = 6 Hz, $\underline{CH_3}(CH_2)_8$)
1.2 to 1.5 (15H, $CH_3\underline{(CH_2)_6}$ - $CH_2CH_2$ and $\underline{CH_3}CH_2OCO$)
1.65 (2H, m, $CH_3(CH_2)_6$ - $\underline{CH_2}$ - $CH_2$)
2.72 (2H, t, J = 8 Hz, $CH_2(CH_2)_7$ - $\underline{CH_2}$)
4.37 (2H, q, J = 7 Hz, $CH_3$- $\underline{CH_2}$ - OCO)
6.60 (1H, s, H in the pyrazole ring)
12.47 (1H, br., NH which disappears by treatment with $D_2O$).

Example 4:

Synthesis of 5-n-nonylpyrazole-3-carboxylic acid:

In the same manner as in Example 2 , 12.0 g (0.045 mol) of ethyl 5-n-nonylpyrazole-3-carboxylate obtained as in Example 3 was hydrolyzed to 5-n-nonylpyrazole-3-carboxylic acid, which was recrystallised from a mixture of acetone and ethanol to provide colourless needle-like crystals melting at 147 to 149°C in an amount of 7.2 g corresponding to a yield of 67.3 %.

Elementary analysis :

found : 65.60 % C; 9.23 % H and 11.82 % N

calcd. as $C_{13}H_{22}O_2N_2$ : 65.51 % C; 9.31 % H and 11.76 % N.

Infrared absorption spectrum

$\nu_{max}^{KBr}$ 3280 $cm^{-1}$ (br. $\nu_{OH}$), 3000 to 2300 $cm^{-1}$ (br. $\nu_{NH_2^+}$) and 1680 $cm^{-1}$ ($\nu_{C=O}$).

Mass spectrum : m/e 238 ($M^+$).

NMR (in $d_6$-DMSO) $\delta$:

0.85 (3H, t, J = 7 Hz, $\underline{CH_3}(CH_2)_8$)

1.2 to 1.5 (12H, br. s, $CH_3\underline{(CH_2)_6}$ $-CH_2CH_2$)

1.65 (2H, m, $CH_3(CH_2)_6$ - $\underline{CH_2}$- $CH_2$)

2.61 (2H, t, J = 7 Hz, $CH_3(CH_2)_6$ - $CH_2$- $\underline{CH_2}$)

6.45 (1H, s, H in the pyrazole ring)

7.87 (2H, br. NH and $CO_2H$ which disappear by treatment with $D_2O$).

Example 5:

Synthesis of ethyl 5-n-undecylpyrazole-3-carboxylate:

In the same manner as in Example 3, 74 g of sodium 1-ethoxycarbonyl-3-oxo-1-tetradecenolate was obtained from 5.8 g (0.25 mol) of metallic sodium, 50 g (0.25 mol) of methyl n-undecyl ketone and 36.5 g (0.25 mol) of diethyl oxalate. Then, in the same

manner as in Example 3, 37 g (0.11 mol) of the thus obtained sodium tetradecenolate was reacted with 6.5 g (0.13 mol) of hydrazine hydrate to provide crude ethyl 5-n-undecylpyrazole-3-carboxylate, which was recrystallized from n-hexane to provide colourless needle-like crystals melting at 39 to 40°C in an amount of 24.2 g corresponding to a yield of 71.0 %.

Elementary analysis :

found : 67.36 % C; 9.85 % H and 10.79 % N

calcd. as $C_{17}H_{30}O_2N_2$ : 67.63 % C; 9.84 % H and 10.52 % N.

Infrared absorption spectrum

$\nu^{nujol}_{max}$ 3200 to 3100 $cm^{-1}$ ($\nu_{NH}$) and 1722 $cm^{-1}$ ($\nu_{C=O}$).

Mass spectrum : m/e 294 ($M^+$).

NMR (in $CDCl_3$) $\delta$:

0.90 (3H, t, J = 6 Hz, $\underline{CH_3}(CH_2)_{10}$)

1.2 to 1.6 (19H, $CH_3\underline{(CH_2)_8}(CH_2)_2$ and $\underline{CH_3}CH_2OCO$)

1.7 (2H, m, $CH_3(CH_2)_8 - \underline{CH_2} - CH_2$)

2.88 (2H, t, J = 8 Hz, $CH_3(CH_2)_8CH_2 - \underline{CH_2}$)

4.65 (2H, q, J = 7 Hz, $CH_3 - \underline{CH_2} - OCO$

6.98 (1H, s, H in the pyrazole ring)

12.7 (1H, br., NH which disappears by treatment with $D_2O$).

Example 6:

Synthesis of 5-n-undecylpyrazole-3-carboxylic acid:

In the same manner as in Example 2, 10 g (0.03 mol) of ethyl 5-n-undecylpyrazole-3-carboxylate obtained as in Example 5 was hydrolysed to

5-n-undecylpyrazole-3-carboxylic acid, which was recrystallized from ethanol to provide colourless needle-like crystals melting at 151 to 153°C in an amount of 7.8 g corresponding to a yield of 86.3 %.

Elementary analysis :

found : 67.36 % C; 9.85 % H and 10.79 % N

calcd. as $C_{15}H_{26}O_2N_2$ : 67.63 % C; 9.84 % H and 10.52 % N.

Infrared absorption spectrum

$\nu_{max}^{KBr}$ 3270 $cm^{-1}$ (br. $\nu_{OH}$ and $\nu_{NH}$), 3000 to 2300 $cm^{-1}$ (br. $\nu_{NH_2^+}$ and $CO_2H$) and 1710 $cm^{-1}$ ($\nu_{C=O}$).

Mass spectrum : m/e 266 ($M^+$).

NMR (in $d_6$-DMSO) $\delta$:

0.85 (3H, t, J = 7 Hz, $\underline{CH_3}(CH_2)_{10}$)

1.2 to 1.5 (16H, br. s, $CH_3\underline{(CH_2)_8} - CH_2CH_2$)

1.60 (2H, m, $CH_3(CH_2)_8 - \underline{CH_2} - CH_2$)

2.55 (2H, t, J = 7 Hz, $CH_3(CH_2)_8CH_2 - \underline{CH_2}$)

5.20 (2H, br. NH and $CO_2H$ which disappear by treatment with $D_2O$)

6.31 (1H, s, H in the pyrazole ring).

Figures 1 to 6 of the accompanying drawings show the infrared absorption spectra of the pyrazole derivatives prepared in accordance with Examples 1 to 6, the number of each Figure corresponding to the number of the relevant Example.

Example 7:

Preparation of tablets:

A uniform mixture of 200 g of 5-n-heptyl-pyrazole-3-carboxylic acid, 50 g of lactose, 20 g of

potato starch, 15 g of CMC and 10 g of methylcellulose was shaped into particles by introducing the mixture together with an aqueous 50 % ethanolic solution into a tumbling forming machine, and the thus obtained particles were dried using a fluidizing dryer. After drying, the particles were sifted by screening machine provided with a sieve having a sieve opening of 500 microns (32 mesh Tyler standard). The particles which passed through the sieve were mixed with 3 g of talc and 2 g of magnesium stearate and the mixture was pressed in a rotary tabletting machine into tablets each 9 mm in diameter and 300 mg in weight.

Example 8:

Preparation of capsules:

Particles were prepared in the same manner as in Example 7 but using 5-n-nonylpyrazole-3-carboxylic acid instead of 5-n-heptylpyrazole-3-carboxylic acid as in Example 7, and the particles were capsulated in No.1 hard capsules by using a capsulating machine at a rate of 300 mg of the particles/capsule.

CLAIMS

1. A compound for use in a method of treatment of the human or animal body by therapy, characterised in that the compound is of the formula:

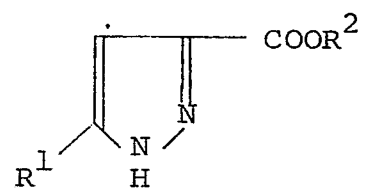

wherein $R^1$ represents an alkyl group of 7 to 11 carbon atoms and $R^2$ represents a hydrogen atom or a lower alkyl group.

2. A compound according to claim 1 for use in treating hyperlipemia.

3. A compound according to claim 1 or 2 wherein said compound is 5-n-heptylpyrazole-3-carboxylic acid.

4. A compound according to claim 1 or 2 wherein said compound is 5-n-nonylpyrazole-3-carboxylic acid.

5. A compound according to claim 1 or 2 wherein said compound is 5-n-undecylpyrazole-3-carboxylic acid.

6. A compound according to claim 1 or 2 wherein said compound is ethyl 5-n-heptylpyrazole-3-carboxylate.

7. A compound according to claim 1 or 2 wherein said compound is ethyl 5-n-nonylpyrazole-3-carboxylate.

8. A compound according to claim 1 or 2 wherein said compound is ethyl 5-n-undecylpyrazole-3-carboxylate.

CLAIMS FOR AUSTRIA

1. A process for preparing a carboxylic acid or alkyl ester thereof of the formula:

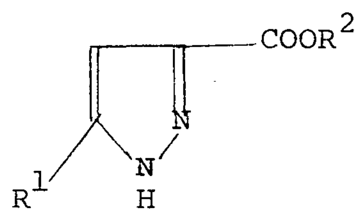

wherein $R^1$ represents an alkyl group of 7 to 11 carbon atoms and $R^2$ represents a hydrogen atom or a lower alkyl group, which process is characterised by reacting a sodium enolate of the formula

$$R^1CO.CH{=}C(ONa)CO_2R^2$$

wherein $R^1$ is as defined herein and $R^2$ is a lower alkyl group, with hydrazine hydrate or hydrazine sulphate.

2. A process according to claim 1 characterised in that an alkyl ester is prepared by reacting the sodium enolate with hydrazine hydrate by heating in a mineral acid under reflux, and if desired the alkyl ester is hydrolysed to the corresponding carboxylic acid.

3. A process according to claim 1 characterised in that a carboxylic acid is prepared by heating the sodium enolate with hydrazine sulphate in an aqueous alcoholic solution of sodium hydroxide.

FIG. 1

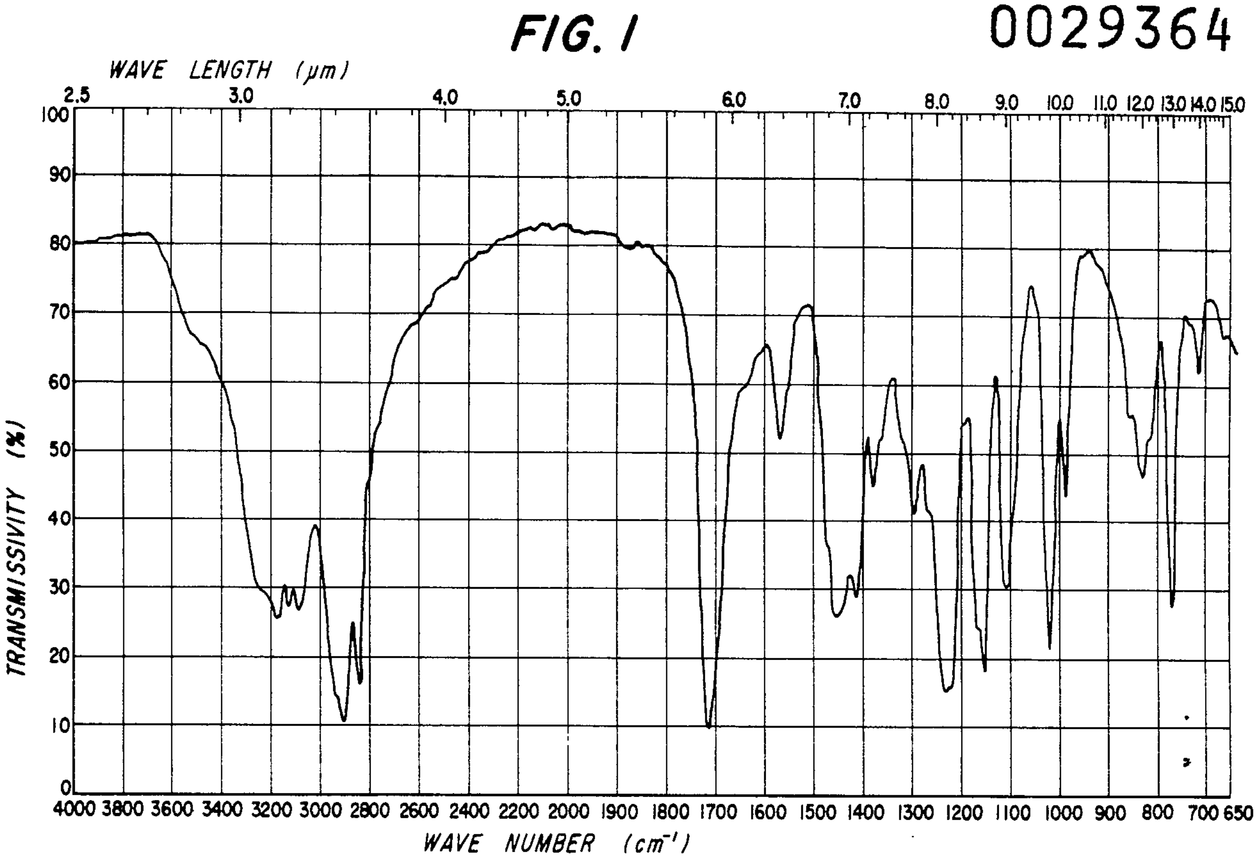
WAVE LENGTH (μm)
2.5 3.0 4.0 5.0 6.0 7.0 8.0 9.0 10.0 11.0 12.0 13.0 14.0 15.0
TRANSMISSIVITY (%)
0 10 20 30 40 50 60 70 80 90 100
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 2

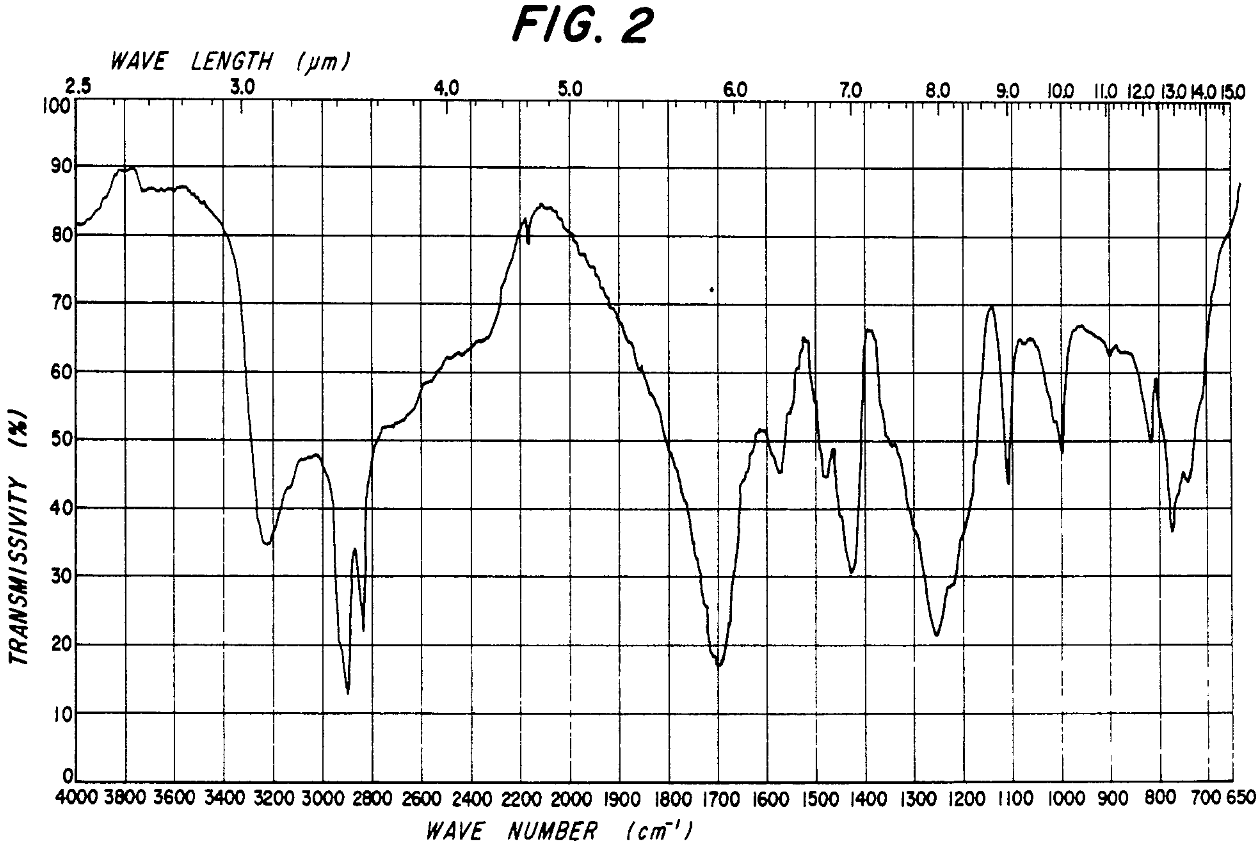
WAVE LENGTH (μm)
2.5 3.0 4.0 5.0 6.0 7.0 8.0 9.0 10.0 11.0 12.0 13.0 14.0 15.0
TRANSMISSIVITY (%)
0 10 20 30 40 50 60 70 80 90 100
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 3

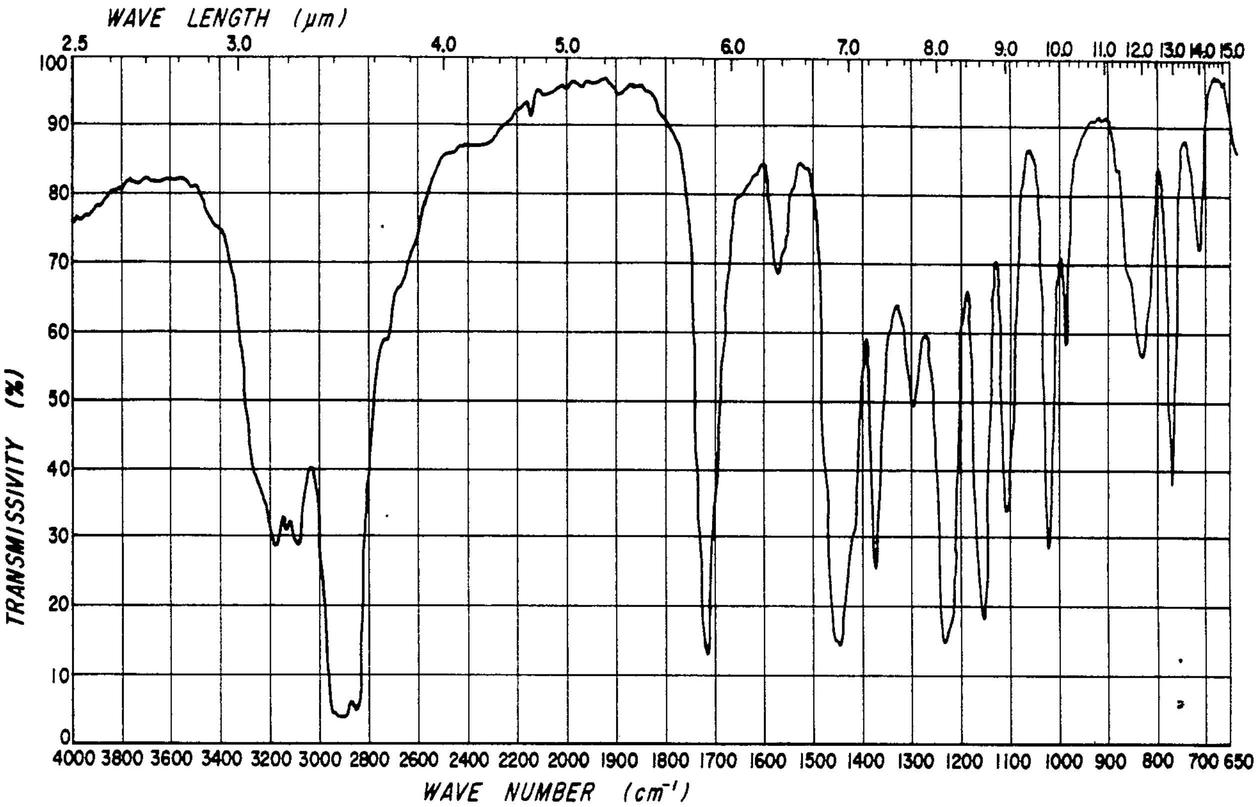

WAVE LENGTH (μm)
2.5
3.0
4.0
5.0
6.0
7.0
8.0
9.0
10.0
11.0
12.0
13.0
14.0
15.0
100
90
80
70
60
50
40
30
20
10
0
TRANSMISSIVITY (%)
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)


FIG. 4

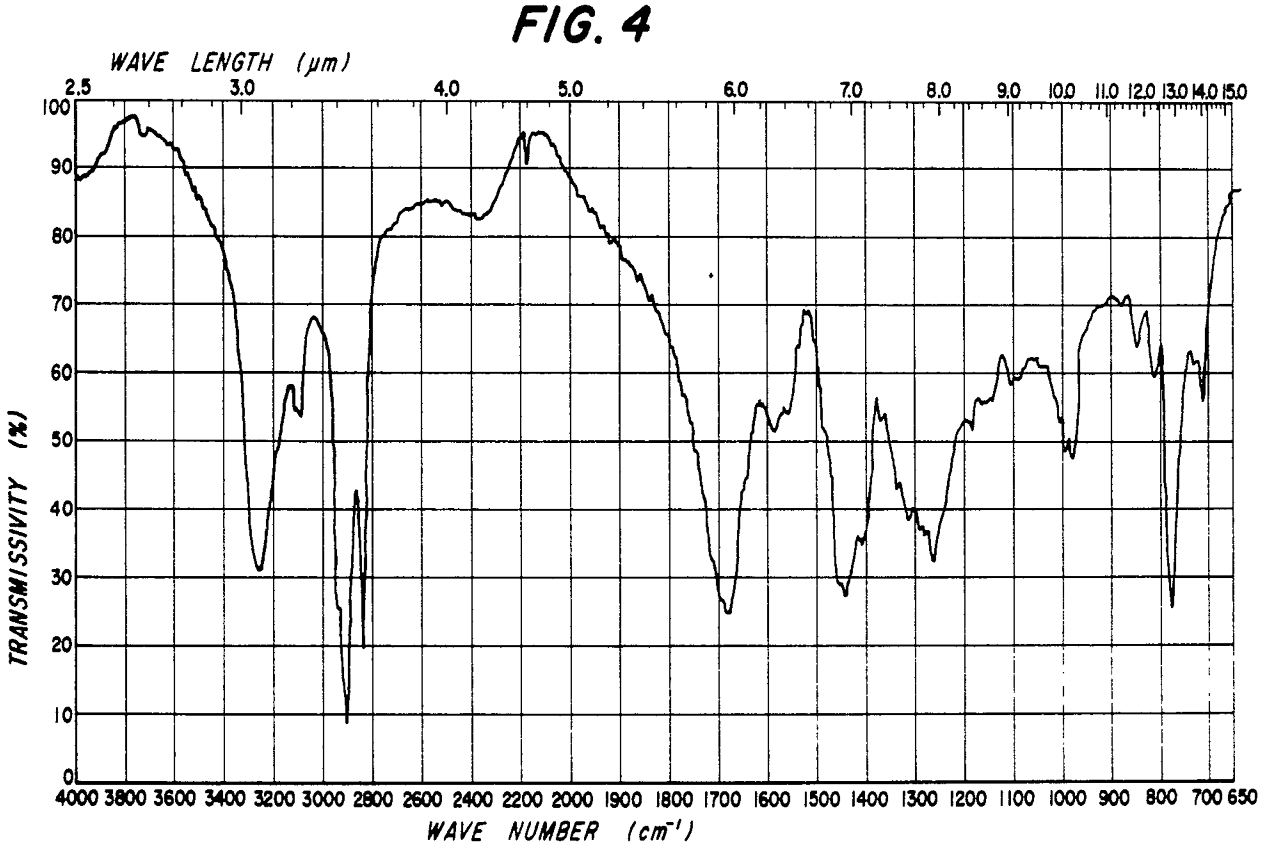

WAVE LENGTH (μm)
2.5
3.0
4.0
5.0
6.0
7.0
8.0
9.0
10.0
11.0
12.0
13.0
14.0
15.0
100
90
80
70
60
50
40
30
20
10
0
TRANSMISSIVITY (%)
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 5

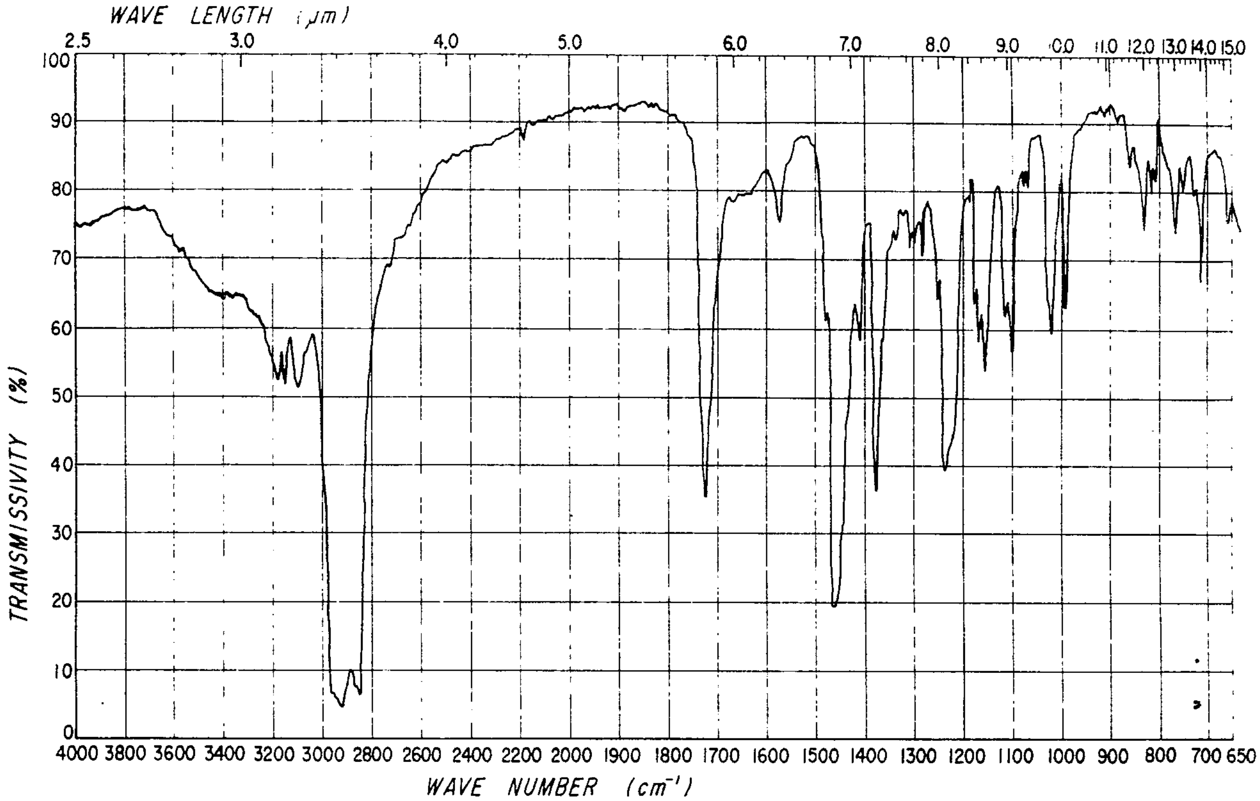
WAVE LENGTH (μm)
2.5 3.0 4.0 5.0 6.0 7.0 8.0 9.0 10.0 11.0 12.0 13.0 14.0 15.0
TRANSMISSIVITY (%)
0 10 20 30 40 50 60 70 80 90 100
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

FIG. 6

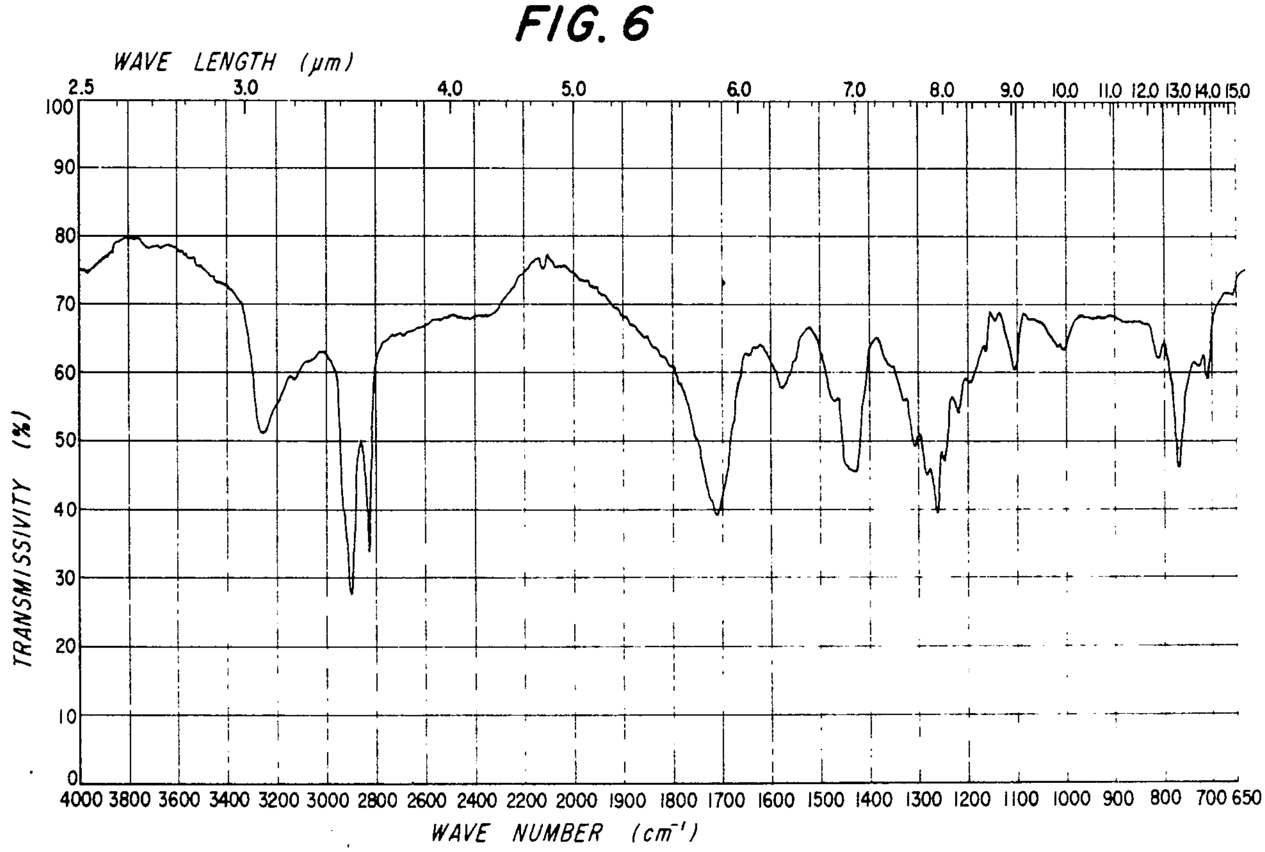
WAVE LENGTH (μm)
2.5 3.0 4.0 5.0 6.0 7.0 8.0 9.0 10.0 11.0 12.0 13.0 14.0 15.0
TRANSMISSIVITY (%)
0 10 20 30 40 50 60 70 80 90 100
4000 3800 3600 3400 3200 3000 2800 2600 2400 2200 2000 1900 1800 1700 1600 1500 1400 1300 1200 1100 1000 900 800 700 650
WAVE NUMBER (cm⁻¹)

European Patent Office

# EUROPEAN SEARCH REPORT

Application number

0029364

EP 80304104.8

## DOCUMENTS CONSIDERED TO BE RELEVANT

| Category | Citation of document with indication, where appropriate, of relevant passages | Relevant to claim |
|---|---|---|
| | GB - A - 1 048 104 (UPJOHN)<br>+ Totality +<br>-- | 1,2 |
| | US - A - 3 449 350 (WALKER)<br>+ Abstract +<br>-- | 1 |
| | US - A - 3 899 508 (WIKEL)<br>+ Columns 1,2 +<br>---- | 1 |

**CLASSIFICATION OF THE APPLICATION (Int. Cl. 3)**

C 07 D 231/14
A 61 K 31/415

**TECHNICAL FIELDS SEARCHED (Int.Cl. 3)**

C 07 D 231/00
A 61 K 31/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 10-02-1981 | LUX |

EPO Form 1503.1 06.78